Europäisches Patentamt

European Patent Office

Office européen des brevets

(19)

(11) Publication number: **0 196 323 B1**

(12) **EUROPEAN PATENT SPECIFICATION**

(45) Date of publication of patent specification: **15.01.92**  (51) Int. Cl.⁵: **A61M 5/32**

(21) Application number: **85904920.7**

(22) Date of filing: **20.09.85**

(86) International application number:
**PCT/US85/01809**

(87) International publication number:
**WO 86/01729 (27.03.86 86/07)**

(54) **POSITIONABLE TISSUE INTERFACING DEVICE FOR THE MANAGEMENT OF PERCUTANEOUS CONDUITS.**

(30) Priority: **21.09.84 US 653442**

(43) Date of publication of application:
**08.10.86 Bulletin 86/41**

(45) Publication of the grant of the patent:
**15.01.92 Bulletin 92/03**

(84) Designated Contracting States:
**AT BE CH DE IT LI LU NL SE**

(56) References cited:
**FR-A- 2 109 932      US-A- 2 456 257
US-A- 3 856 021      US-A- 4 266 999
US-A- 4 278 092      US-A- 4 405 313
US-A- 4 412 832      US-A- 4 479 795
US-A- 4 484 911      US-A- 4 491 126
US-A- 4 515 593**

(73) Proprietor: **Vitaphore Corporation
1505 O'Brien Drive
Menlo Park, CA 94025(US)**

(72) Inventor: **YAMAMOTO, Ronald Kenichi
1454 Regent Street
Redwood City, CA 94061(US)**
Inventor: **PESOTCHINSKY, Sophia
1178 Hyde Avenue
San Jose, CA 95129(US)**

(74) Representative: **Hughes, Brian Patrick et al
Graham Watt & Co. Riverhead
Sevenoaks, Kent TN13 2BN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 196 323 B1

## Description

### Field of the Invention

This invention relates to a device for the improved management of microbial invasion associated with percutaneous conduits, but more particularly to a design which allows the device to be used in conjunction with an existing conduit and positioned along the length of the conduit to the desired anatomical site for optimal effectiveness.

### Description of the Prior Art

A percutaneous device or conduit is an implement which passes through the skin, allowing the linkage of an intracorporeal organ or cavity with extracorporeal equipment. There exists a wide variety of clinical applications for percutaneous devices. In the facilitation of fluid transport, percutaneous conduits, or catheters, are used to access blood for dialysis, pressure monitoring, or laboratory diagnosis, to deliver drugs or nutritional solutions, and to drain wound exudate. Percutaneous electrical leads are used to allow the monitoring of biopotentials from natural organs or implanted instrumentation, as well as for stimulation of such organs.

Despite the widespread clinical need and usage of percutaneous conduits, the presence of the conduit eventually results in bacterial infection, compromising the health of the patient and forcing conduit removal.

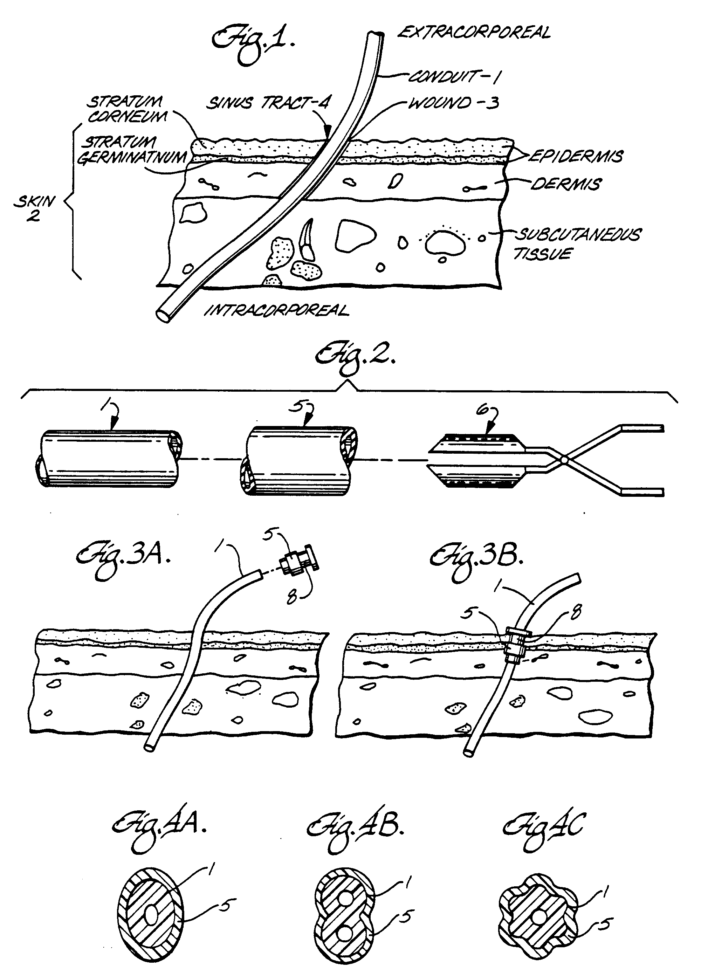

For clarity, Figure 1 depicts the anatomical relationships referred to in the following description of the infection mechanism associated with percutaneous conduits. A conduit 1 is showing spanning the various layers of the skin 2. The portion of the conduit 1 which lies outside the body is referred to as extracorporeal, while that within the body is intracorporeal. Conduit 1 is implanted through a wound in the skin; wound 3 may have been made expressly for the purpose of inserting conduit 1, or it may have been the result of an injury. In either case, a sinus tract 4 is formed by ingrowth of epithelium into the wound. A good review of the biology and failure mechanisms associated with percutaneous devices can be found in CRC Critical Reviews in Bioengineering, Vol. 5, pp. 37-77, 1981, by A.F. von Recum and J. B. Park.

Bacteria that have penetrated beneath the epithelium-conduit interface into underlying tissues find a favorable environment for colonization. The tissues contacting implanted foreign bodies are in a state of chronic inflammation, impairing the normal defense mechanisms against bacterial infection, a basis for the commonly accepted fact that wounds containing foreign bodies are more susceptible to infection. (Williams and Road, Implants in Surgery, 241-244, W. B. Saunders Co., Ltd., 1973.) A classic study demonstrated that while $7.5 \times 10^6$ viable staph aureus organisms were necessary to produce a skin infection, only $3 \times 10^2$ organisms produced an infection in the presence of a silk stitch inserted in the wound. [Elek and Conen, British Journal of Experimental Pathology, 38:573, (1957).] A more recent study has indicated the phagocytosis of bacteria by leukocytes, the secondary defense against bacterial invasion, is impaired in the presence of some foreign materials, at least in vitro. (Borges, L.F., Neurosurgery, 10:55-60, 1982) Typically, once established, bacterial infections around implanted foreign materials cannot be managed, even with massive antibiotic therapy, forcing removal of the implant.

The advance of bacterial colonization along the conduit surface and interfacing tissues may also result in peritonitis when the peritoneal cavity is penetrated by the conduit. Similarly, joint capsule infection may occur when a wound is drained after orthopedic surgery, and septicemia may result when the vascular system is accessed by an intravenous or arterial line. The constant bacterial challenge and threat of infection created by percutaneous conduits forces conduit removal and replacement in alternate areas when possible, and either discontinuation of the function of the conduit or increased risk of infection when not possible.

The bacterial organisms which initiate infections associated with percutaneous conduits are part of the normal flora on the skin, ranging in density from 10 to $10^6$ organisms per square centimeter. Normally these organisms are unable to colonize into deeper tissues and proliferate, as they cannot penetrate the primary barrier against infection, the epidermal layer of the skin. The presence of a percutaneous conduit requires disruption of this barrier, through which bacteria can invade deeper tissues.

The perimeter at the conduit interface does not seal, even after sufficient time for wound healing. The new, healing epithelium, upon reaching the conduit, does not recognize and adhere to the foreign body, but instead, grows down along the conduit in an effort to externalize the foreign body, forming a sinus tract 4, a pathway for bacterial invasion. A recent clinical study has indicated significant association of bacterial colonization of percutaneous catheters and the density of bacteria present on the skin at the exit site, as one would expect from the infection pathway. (Bjornson, et al., Surgery, Vol. 92, No. 4, pp. 720-726, 1982).

The lack of a seal at the conduit to the dermal and epidermal tissues not only allows access for bacteria, but also prevents mechanical loads from being transferred between the tissues and the conduit. The mechanical discontinuity causes a crevice between the tissues and a conduit to open and close when the conduit is mechanically stressed or when the tissues move during normal muscle usage, promoting bacterial invasion.

In order to reduce the ever-present threat of infection associated with percutaneous conduits, devices to improve the tissue interface at the percutaneous area (dermis- and epidermis-conduit interface) have been constructed. The use of porous material which promotes tissue ingrowth in the tissue layers below the epidermis has shown effectiveness in inhibiting epidermal downgrowth (sinus tract formation) and promoting a seal at the epidermal- and dermal-conduit interface. (Lee, U.S. Pat. 3,663,965; Borsany, U.S. Pat. 4,278,092.) However, porous materials with interconnected porosity, such as velours, felts, foams, and sponges, have a disadvantage in that once infected, the material acts as a wick, allowing the bacteria to colonize the pores filled with wound exudate and wound debris, in areas not readily accessible to host defense cells.

Other attempts at combating bacterial infection related to percutaneous conduits involve the application of antibacterial agents, either to the conduit surface (Crossley, U.S. Pat. 4,054,139), or in a fluid reservoir device constructed around the conduit, located in the subcutaneous tissue just below the conduit exit site (Kitrilakis, et al., U.S. Pat. 3,699,956).

Several problems arise when using a tissue interfacing device at the percutaneous interface to help reduce the incidence of infection. The use of devices which have bulky subcutaneous flanges (Faso, U.S. Pat. 4,217,664) or antibacterial fluid reservoirs (Kitrilakis, supra) require surgical manipulation of tissues to properly place the device. If the device has a tissue sealing design using porous materials, such as a velour sleeve, the material should be located close to the dermis-conduit interface. Misplacement in the intracorporeal direction will allow mechanical discontinuity at the critical dermis-to-conduit interface, and also will not allow the epidermis and dermis to grow into the porous material to provide a bacterial seal. Misplacement in the extracorporeal direction will create passageways for bacteria through the pores and immediately introduce external skin bacteria to colonize the porous material.

According to US-A-4266999 which corresponds to the preamble of Claim 1 a cannula/catheter is provided which is suitable for long term or semipermanent through-the-skin function while permitting tissue ingrowth which promotes healing and seals out sources of infection. The catheter is provided with a sheath formed of an elastomeric coating which is glow discharge treated, folded back on itself to form a cuff and surgically implanted and anchored in place. Said sheath resists longitudinal movement along the catheter whereas the cuff portion of the sheath is advanced outwardly by the growth of skin adherent to its periphery while the catheter is maintained in its original position.

Also, when implanting a percutaneous conduit with an attached percutaneous device, or otherwise designated percutaneous area, the length of conduit from the percutaneous exit site to the intracorporeal end is desired to be variable, to allow for the anatomical geometry of the particular patient. Inaccuracies involved in estimating this length may cause the improper location of the percutaneous tissue-interfacing device in the tissue layers of the integuement, the improper location of the intracorporeal end, undesirable slack in the conduit, or required trial fittings to establish the correct length.

The percutaneous tissue-interfacing device of this invention is easily positionable along the conduit length to provide flexibility for optimal device placement during the surgical procedure. Additionally, the ability to be quickly positioned and attached to a pre-existing percutaneous conduit allows the device to be used as an optional measure for patients at high risk for infection.

Summary of the Invention

The invention comprises the combination of a percutaneously inserted conduit and a tissue interface device designed to be placed onto said percutaneous conduit and positioned along the conduit length to a desired anatomical site for effective infection resistance. The invention can be implemented at the time of conduit implantation, or at a later time at the discretion of the clinical practitioner.

Briefly, the invention comprises the combination comprising a percutaneously inserted conduit having an intracorporeal portion extending beneath a skin exit site, and an elastomeric sleeve around the intracorporeal portion of the conduit and resisting longitudinal movement along the conduit, characterised in that the sleeve is located in a single layer on the conduit, has a relaxed inside diameter smaller than the diameter of the conduit, is sufficiently elastic to enable forced dilation so that the sleeve can be enlarged in inside diameter and slipped along the conduit to a desired subcutaneous position after conduit implantation, and has an outer surface which promotes tissue ingrowth.

In one form the inner surface of the sleeve is a resilient silicone rubber, and the outer tissue-ingrowth surface is a sponge-like material The sleeve with elastic properties is dilated with the aid of a special introducer and is then placed over a conduit or catheter-like device. When the introducer is removed, the elastomeric properties of the sleeve material cause its diameter to decrease resulting in radial compression against the conduit, thus securing the sleeve.

The outer, tissue interfacing surface of the device is designed to reduce the incidence of infection related to percutaneous conduits by two basic mechanisms:
(a) promotion of a tissue-to-conduit seal at the skin (epidermis, dermis, and adjacent subcutaneous tissues) to conduit interface, and (b) the use of antibacterial agents incorporated into the device to reduce the possibility of bacteria colonizing the foreign materials (especially during the initial formation of a tissue seal) and advancing along the conduit into deeper tissues.

Description of the Drawings

Fig. 1 shows the anatomical relationship between a percutaneous conduit, the skin and the body surface;
Fig. 2 illustrates the relationship between a conduit, a sleeve and an introducer;
Fig. 3 shows how a sleeve is positioned by an introducer on a conduit;
Fig. 4 shows differently shaped conduits in cross section;
Fig. 5 is a drawing of a conduit with longitudinally placed tissue ingrowth material;
Fig. 6 shows one type of introducer;
Fig. 7 illustrates the use of an introducer of the type shown in Figure 6;
Fig. 8 illustrates an alternative embodiment of an introducer;
Fig. 9 shows one alternative embodiment of the sleeve of Figure 2;
Fig. 10 shows another alternative embodiment of a sleeve; and
Fig. 11 shows yet another embodiment of a sleeve, where the sleeve is wound around the conduit.

Description of the Preferred Embodiments

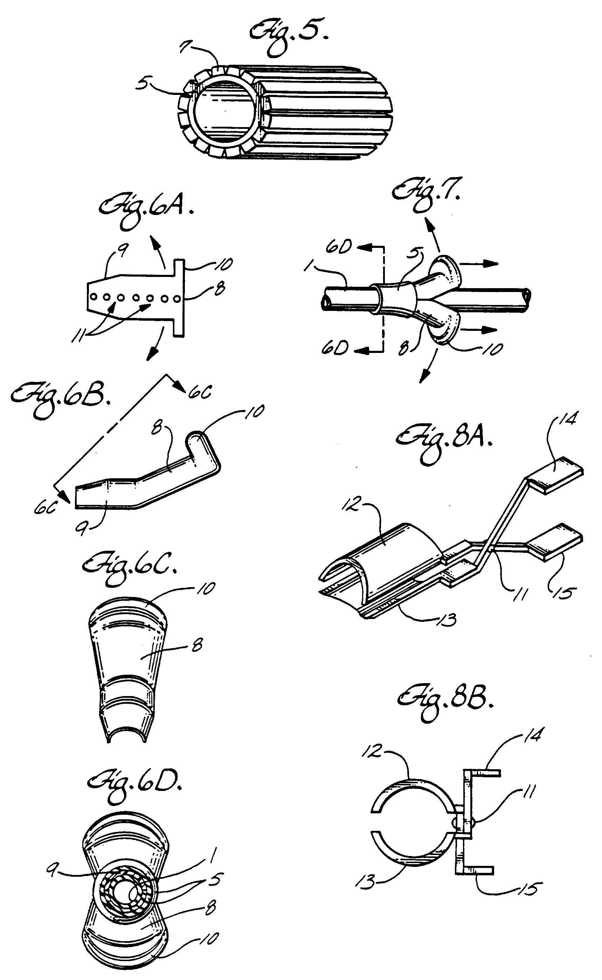

Referring to Figure 2, the preferred embodiment device of the present invention includes a sleeve 5, with an inside diameter less than the outside diameter of a conduit, 1. The term "conduit" is used in a general sense to include both hollow tubular catheters and other types of elongated body-insertable members (either solid or hollow) such as electrical leads.

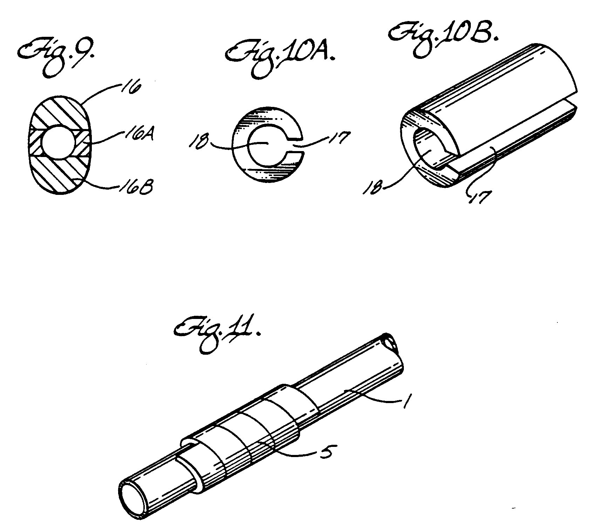

The device is elastically dilated by way of an introducer 6. Introducer 6, which will be described in greater detail below, is inserted into the sleeve 5, and used to expand its diameter. The externally extending conduit end is inserted through the introducer 6 and distended sleeve 5, and the sleeve and introducer are moved longitudinally along this conduit to the desired tissue site. Introducer 6 is then removed, allowing sleeve 5 to return to its normal diameter, leaving the tissue-interfacing sleeve clamped onto the conduit. Sleeve 5 will typically be located in a subcutaneous position, as illustrated in Figure 3B, where sleeve 5 is shown serving to secure a percutaneous conduit.

Figures 3a and 3b illustrates the relationship of conduit 1, sleeve 5 and an alternative embodiment of an introducer 8 (shown in detail below). In Figure 3A the assembled introducer 8 and sleeve 5 are shown as they are placed over the extracorporeal conduit 1 in preparation for positioning. In Figure 3B, sleeve 5 has been positioned with respect to skin 2. Introducer 8, still in place, will be removed, leaving sleeve 5 in place around conduit 1. Although this illustration shows one design of introducer, the same relationships between skin, conduit, sleeve and introducer would obtain were an introducer of a different design used.

Essential to the positionability of the device is the elastic property of the sleeve material. This allows high material strain, corresponding to large device dilation, and use of the stored strain energy to provide a radial clamping force upon release. The high deformability of the elastomeric material also allows sealing of sleeve 5 to the conduit 1, even if the conduit cross-section is ellipsoid (Fig. 4a) or has complex geometry (Fig. 4b, 4c). Any elastomer with negligible stress-relaxation properties and high elastic limit is suitable mechanically. It is also desirable from a physiological viewpoint that the elastic material have minimal inflammatory effect on the surrounding tissue. Several types of medical grade elastomers, such as polydimethyl siloxanes (silicone rubbers) or related polymers, polyurethanes and polyisoprenes are suitable.

Essential to the infection reduction properties of the invention is the outer, tissue interfacing surface of sleeve 5. The surface is desired to express both antibacterial activity and tissue sealing properties. The tissue sealing properties can be conferred by (1) the attachment of porous, tissue ingrowth promoting material, such as woven felts, and velours, textured polymers, and foam or sponge-like materials, (2) the surface texturing of the sleeve material by high energy bombardment or salting out methods; (3) the attachment or incorporation of tissue adhesive biomolecules such as lectins.

The antibacterial properties can be conferred by (1) bulk incorporation of antibacterial agent into the sleeve material, (2) surface coating of antibacterial agent, or (3) attachment of a tissue ingrowth promoting material with incorporated antibacterial activity.

Generally, the invention will consist of a sleeve 5, with antibacterial surface properties conferred by one of the methods previously described. It is also preferred to have tissue adhesive properties without the use of relatively permanent porous tissue ingrowth materials. The use of adhesive biomolecules on the outer surface or biodegradable tissue ingrowth materials such as collagen sponge, or a combination of the two, are preferred, as ease of device removal will not become compromised.

In one embodiment of the present invention, shown in Figure 5, sleeve 5 will have an irregular-surfaced tissue ingrowth material 7, attached placed on the outer surface in longitudinal strips for sufficient radial dilation. The ingrowth of subcutaneous and dermal tissue into the pores will form a seal around the conduit, reducing the sinus tract pathway for bacterial invasion around the catheter. As ingrowth materials with inter-connecting porosity (i.e., velours, felts, foams, sponges) can conduct bacterial infection once established, such materials will incorporate an antibacterial agent to prevent bacterial "wicking." Additionally, it is desirable to use biodegradable materials for the ingrowth layer, so that when catheter removal is indicated, the ingrowth material can be left in situ for resorbtion, thereby alleviating the need for surgical excision. Some potential resorbable materials are collagen, polypeptide, polylactic acid, and polyamino acids. Surface texturing can be used in combination with incorporation of antibacterial activity into or onto the sleeve material as previously described.

The thickness, inside diameter and outside diameter of the sleeve 5 will depend on the outside diameter of the conduit and its radial compliance, the properties of the sleeve material, and the coefficient of friction between the conduit outer surface and the sleeve inner surface.

That is to say, the dimensions of a compliant sleeve material are less critical than in material which is less compliant. Also, the greater the coefficient of friction of either the conduit or sleeve, the less the sleeve will tend to slide longitudinally on the conduit; there will therefore be a correspondingly reduced need for radial compression of the conduit by the sleeve to fix the latter in place.

The thickness of an attached porous ingrowth layer 7 will depend on the outside diameter of the sleeve, the expected loads on the percutaneous interface, the degradation rate of the ingrowth layer if it is biodegradable, and the intended period of clinical use.

Another component of the present invention is an introducer 8, to position the device. Introducer 8 is designed to dilate sleeve 5 to allow its positioning over conduit 1. The following examples are given for illustrative purposes. Figures 6 and 7 show a cylindrical introducer 8 with a taper 9 at one end, a flange 10 on the other end, and perforation lines 11 along the length of the introducer radially opposed to each other. The perforation lines enable the introducer to be split into two sections either before installation in the sleeve, or during removal from the sleeve.

Introducer 8 is preferably manufactured in two separate pieces, one of which is shown in end and edge views in Figures 6b and 6c respectively. In using this introducer 8, the two halves are placed together as shown in Figure 6d. The tapered end 9 of introducer 8 is then pushed into sleeve 5, dilating it, and the assembly of introducer 8 and sleeve 5 is pushed onto conduit 1 as shown in Figure 7. When sleeve 5 is correctly positioned, the two halves of introducer 8 are pulled apart, causing its removal from within sleeve 5. An advantage of the two-piece design is that introducer 8 may be easily removed from the site of conduit implantation without threading the length of of the conduit through the introducer. Introducer 8 may be formed of a plastic material or of metal, but it is preferred that the material be slightly flexible. An advantage of using a plastic material is that introducer 8 may be both sterile and disposable.

An alternate embodiment of an introducer as shown in Figures 8A and 8B using two components joined at a pivot 11, would allow the sleeve to be placed over the split cylinder sections, 12 and 13, and dilated when pressure is applied to the actuation pads 14 and 15. The introducer and sleeve is placed over the catheter or conduit, and brought to the desired location. The pressure on the actuation pads is released as the introducer is pulled away from the sleeve, leaving the sleeve on the conduit.

Any rigid polymer which is readily sterilizable and moldable would be suitable for the construction of the introducer. Some examples are polyethylene, polypropylene, and polysulfone. An introducer design such as described in Figures 6, 7 and 8 is not permanently deformed during use, and can be reused. A variety of metals and rigid polymers are suitable for its construction.

## Alternate Embodiments of the Invention

Alternative sleeve-like designs utilizing elastic components to provide residual clamping force for fixation are possible, combined with the tissue sealing components and the incorporation of antibacterial activity as previously described.

A composite device design utilizing elastomeric components 16, in Figure 9, to provide positionability of the device is a simple modification although device complexity is increased. Such a sleeve may be formed in two parts: an elastomeric region 16A and a non-distensible region 16B, bonded to elastomeric region 16A.

A sleeve design with a "C" shaped cross-section, or similar, when deformed (Figure 10), could be placed over a conduit without high dilation through the opening 17, to the lumen 18. Release of the device will allow elastic return to apply a clamping force, although not as efficiently as an integral, cylindrical sleeve. This embodiment of the present invention can be used with the introducers 6 or 8 already described.

A design shown in Figure 11 which consists of a strip of material wound into a helical sleeve could be deformed and placed around a catheter-like tube without high material deformation, yet utilizing the deformation to provide a residual clamping force, although the magnitude of the force will not be as great as in the designs of Figures 2, 3, 4, and 5. The design of the embodiment shown in Figure 11 does not require use of an introducer.

Finally, it should be emphasized that these designs may also be used to secure conduits in wholly intracorporeal sites, as for example when electrical leads need to be implanted in regions of high mobility such as joints, blood vessels, the heart, or in the bowel.

While the present invention has been disclosed in terms of a number of specific embodiments, these embodiments are not intended to limit the scope of the invention as claimed hereinafter.

## Claims

1. The combination comprising a percutaneously inserted conduit (1) having an intracorporeal portion extending beneath a skin exit site, and an elastomeric sleeve (5) around the intracorporeal portion of the conduit and resisting longitudinal movement along the conduit, characterised in that the sleeve is located in a single layer on the conduit, has a relaxed inside diameter smaller than the diameter of the conduit, is sufficiently elastic to enable forced dilation so that the sleeve can be enlarged in inside diameter and slipped along the conduit to a desired subcutaneous position after conduit implantation, and has an outer surface which promotes tissue ingrowth.

2. A combination as claimed in claim 1, characterised in that the surface of the sleeve has been impregnated with a substance having antibacterial properties.

3. A combination as claimed in claim 1, characterised in that only a portion of the circumference of the sleeve is distensible.

4. A combination as claimed in claim 1, characterised in that the circumference of the sleeve is incomplete.

5. A combination as claimed in claim 1, characterised in that the sleeve is formed as a helical coil to be wound around the conduit.

6. A combination as claimed in claim 1, characterised in that the tissue-ingrowth-promoting surface of the sleeve is provided by a material secured to the sleeve outer surface.

7. A combination as claimed in claim 6, characterised in that the tissue-ingrowth material is impregnated with a substance having antibacterial properties.

8. A combination as claimed in claim 6 or claim 7, characterised in that the material attached to the outer surface of the sleeve is selected from the group consisting of velour, felt, foam, sponge, collagen, polylactic acid, polyamino acid and polypeptide.

9. A combination as claimed in claim 1, characterised in that the inside diameter of the sleeve is constant along the length of the sleeve.

## Revendications

1. Combinaison comprenant un conduit (1) inséré à travers la peau et ayant une partie intracorporelle qui s'étend au-dessous de son point de sortie de la peau, et un manchon en élastomère (5) placé autour de la partie intracorporelle du conduit et résistant à un déplacement longitudinal le long du conduit, caractérisée en ce que le manchon est placé en une seule couche sur le conduit, en ce qu'il a, à l'état relâché, un diamètre intérieur plus petit que le diamètre du conduit, en ce qu'il est suffisamment élastique pour subir une dilatation forcée, de sorte que son diamètre intérieur puisse être agrandi et qu'il puisse être glissé le long du conduit vers une position sous-cutanée désirée après l'implantation du conduit, et en ce qu'il a une surface extérieure qui favorise la croissance des tissus.

2. Combinaison selon la revendication 1, caractérisée en ce que la surface du manchon a été imprégnée d'une substance ayant des propriétés antibactériennes.

3. Combinaison selon la revendication 1, caractérisée en ce qu'une partie seulement de la circonférence du manchon est dilatable.

4. Combinaison selon la revendication 1, caractérisée en ce que la circonférence du manchon est incomplète.

5. Combinaison selon la revendication 1, caractérisée en ce que le manchon est réalisé sous la forme d'un enroulement en spirale destiné à être enroulé autour du conduit.

6. Combinaison selon la revendication 1, caractérisée en ce que la surface favorisant la croissance des tissus est fournie par une matière fixée à la surface extérieure du manchon.

7. Combinaison selon la revendication 6, caractérisée en ce que la matière favorisant la croissance des tissus est imprégnée d'une substance ayant des propriétés antibactériennes.

8. Combinaison selon la revendication 6 ou 7, caractérisée en ce que la matière fixée à la surface extérieure du manchon est choisie dans le groupe constitué par du velours, du feutre, de la mousse, de l'éponge, du collagène, un acide polylactique, un polyamino-acide et un polypeptide.

9. Combinaison selon la revendication 1, caractérisée en ce que le diamètre intérieur du manchon est constant le long de la longueur du manchon.

**Patentansprüche**

1. Kombination, bestehend aus einer perkutan eingesetzten Leitung (1) mit einem sich unterhalb einer Hautaustrittsstelle erstreckenden im Körper liegenden Bereich und einer elastomeren Hülse (5) um den im Körper liegenden Bereich der Leitung, die gegenüber einer Längsbewegung entlang der Leitung gesichert ist, dadurch gekennzeichnet, daß die Hülse in einer einzigen Schicht auf der Leitung ange-ordnet ist, einen entspannten Innendurchmes-ser besitzt, der kleiner ist als der Durchmesser der Leitung, in der Weise elastisch ist, daß eine zwangsweise Aufweitung ermöglicht wird, derart, daß die Hülse im Innendurchmesser vergrößert und entlang der Leitung in eine gewünschte subkutane Position nach der Leitungsimplantation verschoben werden kann, und eine äußere Oberfläche aufweist, die einem Gewebeeinwachsen förderlich ist.

2. Kombination nach Anspruch 1, dadurch gekennzeichnet, daß die Oberfläche der Hülse mit einer Substanz imprägniert worden ist, die antibakterielle Eigenschaften besitzt.

3. Kombination nach Anspruch 1, dadurch gekennzeichnet, daß nur ein Bereich des Umfangs der Hülse dehnbar ist.

4. Kombination nach Anspruch 1, dadurch gekennzeichnet, daß der Umfang der Hülse unvollständig ist.

5. Kombination nach Anspruch 1, dadurch gekennzeichnet, daß die Hülse als eine schraubenlinienförmige Spule ausgebildet ist, die um die Leitung herumzuwickeln ist.

6. Kombination nach Anspruch 1, dadurch ge-kennzeichnet, daß die dem Gewebeeinwach-sen förderliche Oberfläche der Hülse von einem Material gebildet ist, daß an der äußeren Oberfläche der Hülse festgelegt ist.

7. Kombination nach Anspruch 6, dadurch ge-kennzeichnet, daß das Gewebeeinwachsmateri-al mit einer Substanz mit antibakteriellen Eigenschaften imprägniert ist.

8. Kombination nach Anspruch 6 oder Anspruch 7, dadurch gekennzeichnet, daß das an der äußeren Oberfläche der Hülse angebrachte Material aus der aus Velours, Filz, Schaum, Schwamm, Collagen, Polymilchsäure, Polyaminsäure und Polypeptid bestehenden Gruppe ausgewählt ist.

9. Kombination nach Anspruch 1, dadurch gekennzeichnet, daß der Innendurchmesser der Hülse auf der Hülsenlänge konstant ist.

Fig.1.

EXTRACORPOREAL

CONDUIT–1

SINUS TRACT–4

WOUND –3

STRATUM
CORNEUM

STRATUM
GERMINATNUM

EPIDERMIS

DERMIS

SKIN
2

SUBCUTANEOUS
TISSUE

INTRACORPOREAL

Fig.2.

1

5

6

Fig.3A.

5

1

8

Fig.3B.

1

5

8

Fig.4A.

1

5

Fig.4B.

1

5

Fig.4C

1

5

8

Fig.5.

Fig.6A.

Fig.7.

Fig.6B.

Fig.8A.

Fig.6C.

Fig.6D.

Fig.8B.

Fig.9.

Fig.10A.

Fig.10B.

Fig.11.